# EUROPEAN PATENT SPECIFICATION

(11) **EP 3 950 047 B1**
(45) Date of publication and mention of the grant of the patent: **16.10.2024**
(21) Application number: 21187863.2
(22) Date of filing: 27.07.2021
(51) Int. Cl.: A61N 1/05, H01R 13/405, H01R 24/58, H01R 43/24, B29C 45/14, A61N 1/375, B29K 63/00, B29K 75/00, B29K 81/00, B29K 83/00, B29K 71/00

(54) **LEAD CONNECTOR WITH ASSEMBLY FRAME AND METHOD OF MANUFACTURE**
LEITUNGSVERBINDER MIT MONTAGERAHMEN UND VERFAHREN ZUR HERSTELLUNG
CONNECTEUR DE FIL AVEC CADRE D'ASSEMBLAGE ET PROCÉDÉ DE FABRICATION

(30) Priority: 27.07.2020 US 202063057033 P
(43) Date of publication of application: 09.02.2022
(73) Proprietor: Heraeus Medical Components, LLC, St. Paul, MN 55127 (US)
(72) Inventor: Schuster, Paul, St. Paul, 55127 (US); West, Jonathan, St. Paul, 55127 (US); Locke, Benjamin, St. Paul, 55127 (US)
(74) Representative: Maiwald GmbH

(56) References cited:
- US-A1- 2014 107 751
- US-A1- 2014 213 118
- US-A1- 2014 309 719
- US-A1- 2015 165 217

## Description

### TECHNICAL FIELD

An implantable medical lead typically includes a tubular-shaped main lead having one or more conductors or coils to sense or provide stimulative biologic, electrical signals, and a lead connector coupled to one end of the main lead. In one embodiment, the lead connector is, inturn, configured to electrically and mechanically plug into and couple to a header or connector bore of a pacemaker, implantable cardioverter defibrillator ("ICD"), or other type of pulse generator.

### BACKGROUND

One type of implantable medical lead includes an IS4/DF4 lead connector, which is a standardized lead connector having an injection molded, reaction injection molded (RIM) or potted, cylindrical body (typically of a thermoplastic, or thermoset material). The connector body has a proximal end configured to connect into a header of an active implantable device of some type, and a distal end configured to connect to the conductors/coils within the main lead. Such lead connectors have multiple electrical contacts in the form of contact rings which are spaced along and are flush with a surface of the connector body. Lead connectors may also include a pin contact extending from the proximal end. A conductor typically extends through the lead body from each contact ring and projects from the distal end of the molded body so as to provide a connection point for the conductors of the main lead. Similarly, a main body pin may extend along a central axis of the lead connector from the pin contact at the proximal end and also project from the distal end of the molded body.

Conventional practices for the manufacture of lead connectors include an injection molding process, or a reaction injection molding process, or liquid silicone molding process, or a potting process wherein the ring connectors, conductive pins, and the central pin/pin contact (if being employed) are arranged within a mold cavity. A thermoplastic material, or other suitable material, is then injected into the mold cavity to over-mold the conductive pins, ring connectors, and main body pin to form the cylindrical body of the lead connector.

US 2015/0165217 A1 discloses an implantable lead connector assembly including contact rings, and bulk of insulation, which includes sealing surfaces and a shank defining a distal end of the bulk. One or more conductor pins extend within the bulk and have distal ends protruding distally therefrom to be exposed alongside the shank; and an inner surface of each contact ring may have a proximal end of a corresponding conductor pin coupled thereto. US 2014/107751 A1 relates to a method for constructing a plug for an electrical connection to a multipolar lead for an active implantable medical device. A method of manufacturing a lead connector for an implantable medical device is described in US 2014/0213118 A1. US 2014/0309719 A1 concerns a lead end containing a slotted member, which can have a plurality of slots extending along at least a portion of the length of the slotted member, each of the slots having a respective positioning feature, the plurality of slots having a plurality of positioning features at different longitudinal positions along the length of the slotted member.

Tight tolerances are required for the safe and effective performance of lead connectors, including IS4/DF4 connectors. However, the injection molding process presents many challenges and shortcomings that make maintenance of such tight tolerance difficult to meet and which can result in high production costs and low manufacturing yields. For these and other reasons, there is a need for the present embodiments.

### BRIEF DESCRIPTION OF THE DRAWINGS

Figure 1 illustrates a perspective view of a lead connector according to one embodiment.
Figure 2 illustrates a side view of the lead connector depicted in Figure 1, according to one embodiment.
Figure 3 illustrates a perspective view illustrating portions of a lead connector in a stage of assembly prior to over-molding, according to one embodiment.
Figure 4 illustrates a side view of illustrating portion of the lead connector depicted in Figure 3, according to one embodiment.
Figures 5-7 illustrate perspective views of an assembly frame, according to one embodiment.
Figure 8 illustrates an end view of an assembly frame, according to one embodiment.
Figure 9 illustrates an exploded view of a ring-pin assembly, according to one embodiment.
Figure 10 illustrates a perspective view of a ring-pin assembly, according to one embodiment.
Figure 11 illustrates a ring-pin assembly in a mold cavity, according to one embodiment.
Figure 12 illustrates a method of forming a lead connector, according to one embodiment.
Figures 13A and 13B illustrate perspective views of an assembly frame, according to one embodiment.
Figure 14 illustrates an exploded view of a ring-pin assembly, according to one embodiment.
Figure 15 illustrates an exploded view of a ring-pin assembly, according to one embodiment.
Figures 16A-16B illustrate perspective views of an assembly frame, according to one embodiment.

### DETAILED DESCRIPTION

According to the present invention a method of manufacturing a lead connector 20 for an implantable device as defined in claim 1 is provided. According to a further aspect of the present invention a lead connector 20 for an implantable device as defined in claim 6 is provided. According to still a further aspect of the present invention a ring-pin assembly 70 as defined in claim 11 is provided.

In the following detailed description, reference is made to the accompanying drawings which form a part hereof, and in which is shown by way of illustration specific examples in which the disclosure may be practiced. It is to be understood that other examples may be utilized and structural or logical changes may be made without departing from the scope of the present disclosure. It is to be understood that features of the various examples described herein may be combined, in part or whole, with each other, unless specifically noted otherwise.

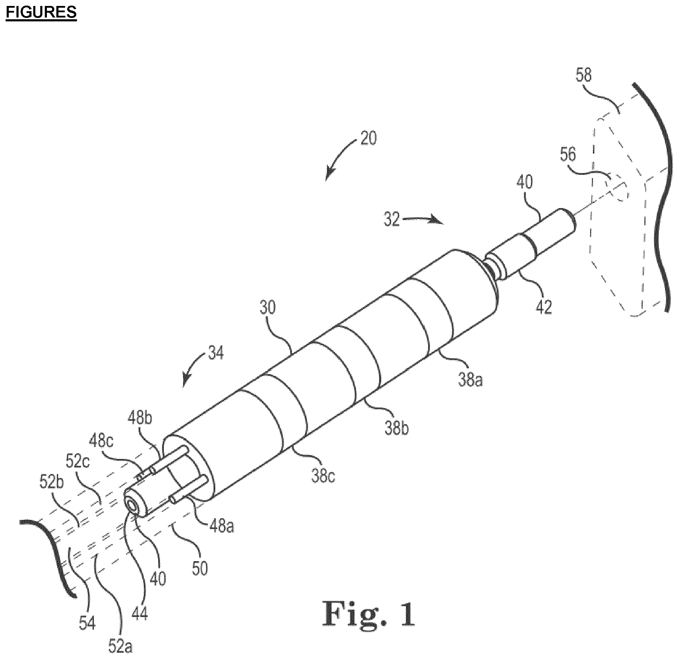

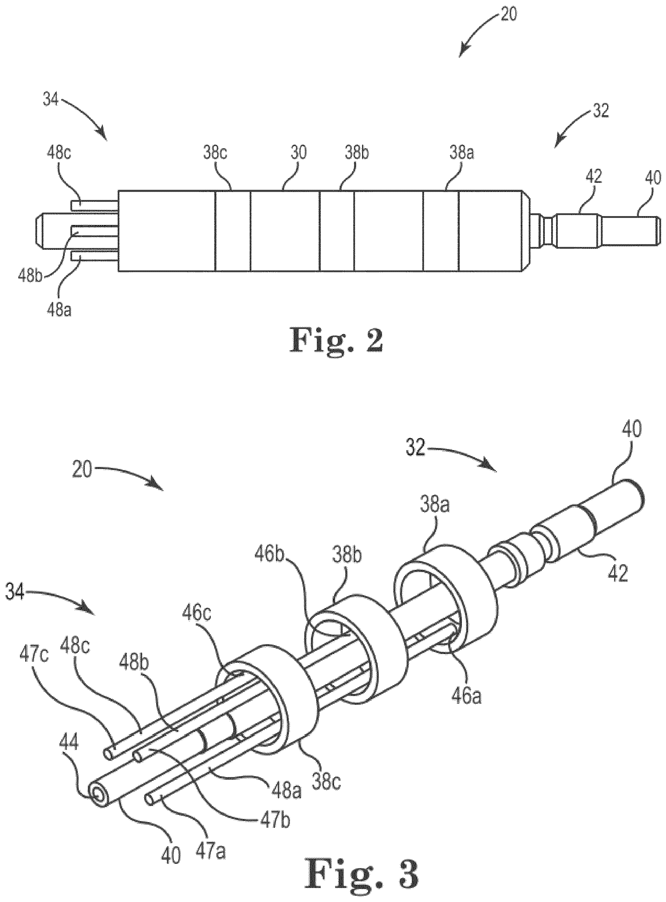

Figure 1 is a perspective view, and Figure 2 is a side view of an example of a lead connector 20 for an implantable medical device. Lead connector 20 can be any of a variety of implantable leads, including, for example, an IS4/DF4 lead connector. Lead connector 20 includes a cylindrical body 30 having a proximal end 32 and a distal end 34 according to one embodiment.

In one embodiment, lead connector 20 includes first-third ring contacts 38a, 38b, 38c, disposed in a spaced-apart fashion along a longitudinal dimension of body 30, and a central pin 40 axially extending from proximal end 32 having a pin contact 42. First-third ring contacts 38a-38c are imbedded in and have a same outer diameter as body 30 so as to provide cylindrical body 30 with a uniform circumferential surface. Body 30 may be formed of an electrically non-conductive polymer material (e.g. polyurethane, polyetheretherketone (PEEK), polysulfone, etc.), epoxy, liquid silicone rubber, or any other suitable type of electrically non-conductive material.

In one embodiment, first-third conductive pins 48a, 48b, and 48c axially extend through body 30 respectively from first-third ring contacts 38a, 38b and 38c, and project from distal end 34 of body 30. In one embodiment, first-third conductive pins 48a-48c are generally rigid wires that form first-third conductive pins 48a-48c. Similarly, central pin 40 extends axially through body 30 and projects from distal end 34, with central pin 40 defining a central lumen 44. In other embodiments, central pin 40 may extend axially through body 30, but project from distal end 34 on the outer periphery, such as with first-third conductive pins 48a-48c.

In one embodiment, lead connector 20 is configured to be coupled to a flexible implantable lead. For example, lead connector 20 can be coupled at its distal end 34 to a flexible implantable lead 50 (illustrated in dashed lines in Figure 1). In one embodiment, first-third conductive pins 48a-48c serve as a contact point to which conductors 52a, 52b, and 52c of flexible implantable lead 50 are electrically and mechanically connected, such as by laser welding, for example, to thereby place lead conductors 52a-52c in electrical communication with first-third ring contacts 38a-38c. Lead 50 may also include a central conductor 54 which is in electrical communication with pin contact 42 via central lumen 44. In one embodiment, conductors 52a-52c and central conductor 54 extend through lead 50 to corresponding coil or sensor.

In one embodiment, lead connector 20 is configured at its proximal end 32 to be coupled to a device. For example, in one embodiment, lead connector 20 is configured to be inserted into a receptacle or bore of 56 of header of a pulse generator 58 (illustrated in dashed lines in Figure 1) of some type, such as a pacemaker or implantable cardioverter defibrillator ("ICD"), for example. Complementary contacts within pulse generator contact ring contacts 38a-38c and pin contact 42, thereby placing pulse generator 58 in electrical communication with sensors and/or coils associated with lead 50.

It is noted that while lead connector 20 has been described with an example primarily in the context of an IS4/DF4 lead connector, one skilled in the art understands that the use of an assembly frame described below, as well as manufacturing techniques described herein, are applicable to other types of lead connectors as well. Accordingly, the features of lead connector 20, including as assembly frame, and methods of manufacture described, herein should not be interpreted as being limited to only IS4/DF4 lead connectors.

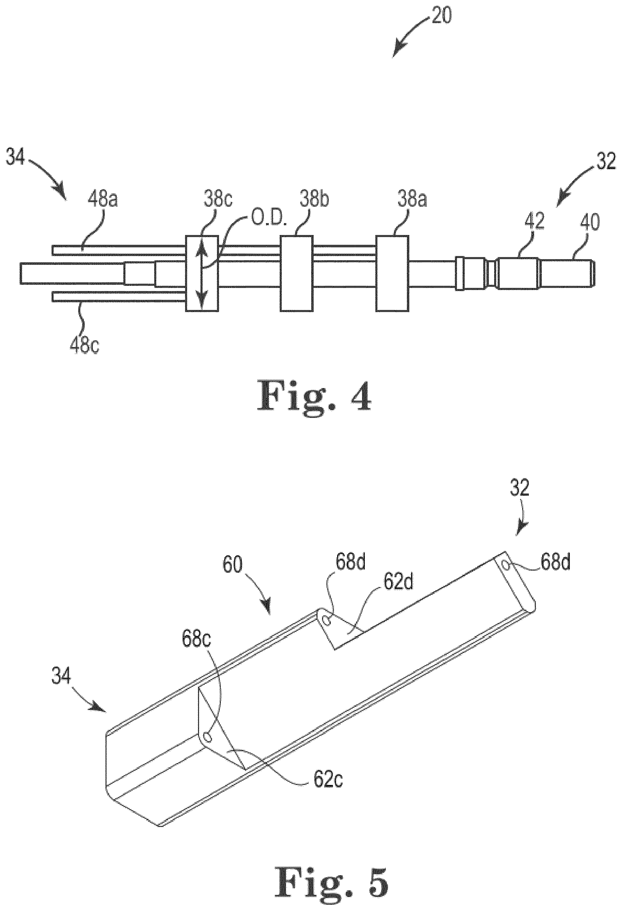

Figures 3 and 4 are respective perspective and side views illustrating portions of lead connector 20, prior to being over-molded to form body 30, according to one embodiment. As illustrated, prior to the molding process for forming body 30, first-third conductive pins 48a, 48b, and 48c are respectively connected to an inner surface of corresponding first-third ring contact 38a, 38b, 38c, such as by laser welding, or soldering, for example, to form first-third ring-pin subassemblies 38a/48a, 38b/48b, 38c/48c.

In one embodiment, each of first-third conductive pins 48a, 48b, and 48c respectively have proximal ends 46a, 46b, and 46c and distal ends 47a, 47b, and 47b. In one embodiment, the proximal ends 46a, 46b, and 46c are respectively fixed to ring contacts 38a, 38b, 38c, while the distal ends 47a, 47b, and 47b each extend back toward the distal end 34 of lead connector 20.

In one embodiment, first-third ring-pin subassemblies 38a/48a, 38b/48b, 38c/48c are configured for "nesting", such that first conductive pin 48a extends from its proximal end 46a at first ring 38a back through second and third rings 38b and 38c toward the distal end 34 of lead connector 20. Similarly, second conductive pin 48b extends from its proximal end 46b at second ring 38b back through third ring 38c toward the distal end 34 of lead connector 20. Third conductive pin 48c extends from its proximal end 46c at third ring 38c back toward the distal end 34 of lead connector 20. In one embodiment, central pin 40 is also arranged so as to extend through all three ring contacts 38a-38c from proximal end 32 to distal end 34.

In one embodiment, it is important that conductive pins extending through rings to which they are not coupled do not inadvertently make contact with these rings. For example, while first conductive pin 48a is coupled to first ring 38a, it cannot make contact with second and third rings 38b and 38c as it extends toward the distal end 34 of lead connector 20. Touching another ring can cause an electrical short. Even getting too near a ring can leave the device susceptibility to electrical arcing.

In some designs, an insulative coating is added to each conductive pin or wire and it exends through adjacent rings in order to avoid inadvertant electrical contact with rings. Similarly, an insulating sleeve can be added to each wire or pin. Both processes, however, add complexity and cost to the manufacturing of a lead connector. For example, a coated wire must be ablated on each of the proximal and distal ends to provide electrical conductivity where it is needed (at connection points). Furthermore, adding individual insulating tubing to each wire is time consuming and adds multiple parts. All of this adds to manufacturing time and cost.

Figures 5-8 illustrate various perspective views and an end view of assembly frame 60 of lead connector 20 in accordance with one embodiment. In one embodiment, assembly frame 60 includes first-third steps 62a, 62b and 62c. In one embodiment, assembly frame 60 includes first-fourth openings 68a, 68b, 68c, and 68d, each extending the length of the assembly frame 60. In one embodiment, first-fourth openings 68a, 68b, 68c, and 68d, are configured as cylindrical lumens with a diameter that accommodates conductive pins 48a, 48b, and 48c. Proximal end 32 and distal end 34 of assembly frame 60 correlate with that of lead connector 20. In one embodiment, assembly frame 60 assists in the assembly of lead connector 20 and overcomes many of the disadvantages of prior systems.

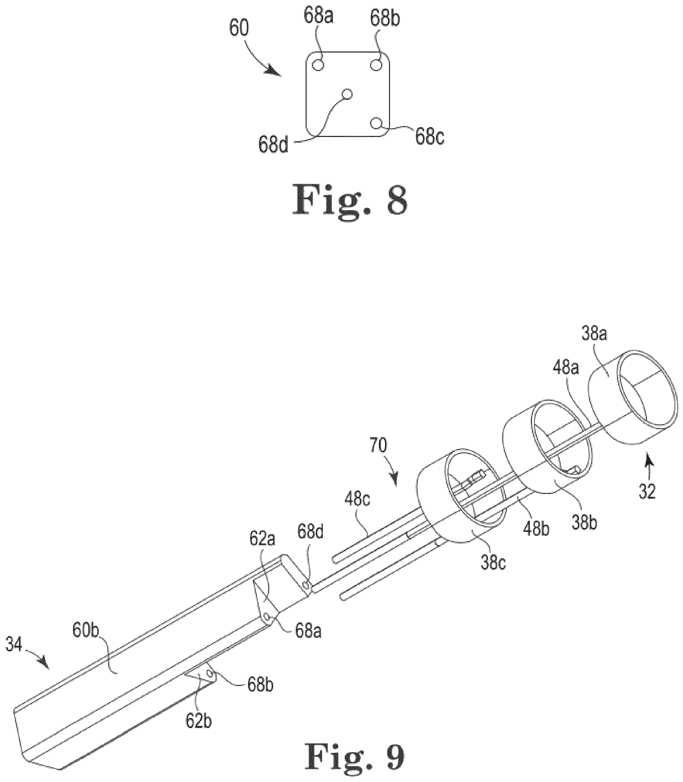

In one embodiment, during the assembly of lead connector 20, each first-third ring-pin subassemblies 38a/48a, 38b/48b, 38c/48c are individually assembled onto assembly frame 60 to form a ring-pin assembly 70. Figure 9 illustrates an exploded view of a partially assembled ring-pin assembly 70 for forming lead connector 20, where each of first-third ring-pin subassemblies 38a/48a, 38b/48b, 38c/48c are adjacent assembly frame 60.

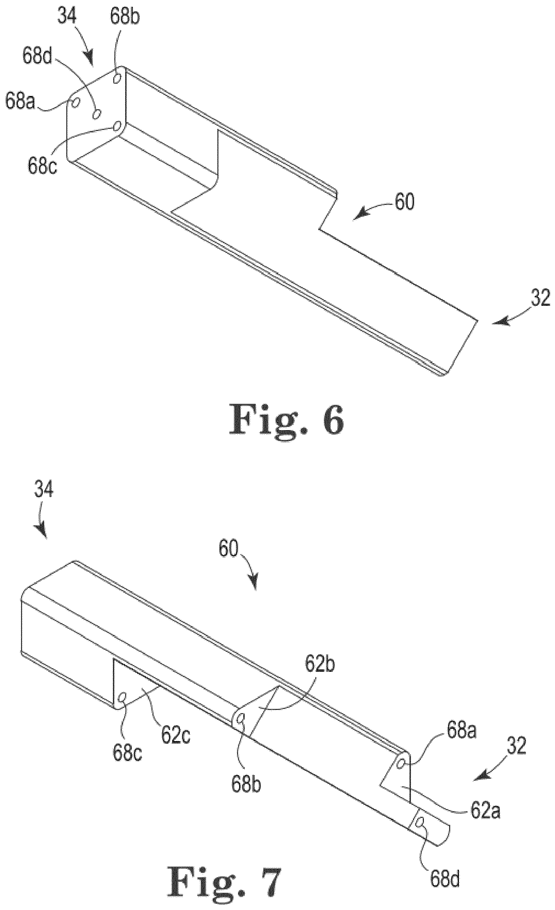

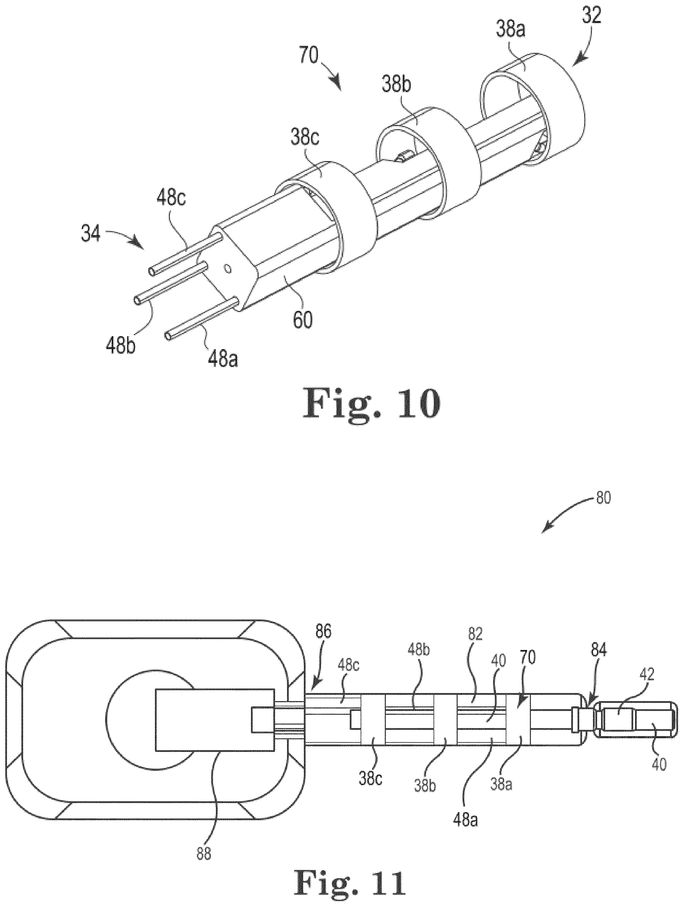

In one embodiment, third ring-pin subassembly 38c/48c is placed over assembly frame 60 by sliding third conductive pin 48c into third opening 68c (not visible in Figure 9, but illustrated in Figures 7-8) and sliding third ring 38c over assembly frame 60 until it engages third step 62c (not visible in Figure 9, but illustrated in Figure 7). Next, second ring-pin subassembly 38b/48b is placed over assembly frame 60 by sliding second conductive pin 48b into second opening 68b and sliding second ring 38b over assembly frame 60 until it engages second step 62b. Finally, first ring-pin subassembly 38a/48a is placed over assembly frame 60 by sliding first conductive pin 48a into first opening 68a and sliding first ring 38a over assembly frame 60 until it engages first step 62a. In one embodiment, central pin 40 is inserted through fourth opening 68d so that it extends out of assembly frame 60 on both proximal end 32 and distal end 34. Fully assembled ring-pin assembly 70 is illustrated in Figure 10.

In one embodiment, assembly frame 60 is configured with first-third steps 62a, 62b and 62c being spaced along its longitudinal dimension such that when a respective ring 38a-48c is placed adjacent the step, the ring will be situated in a desired longitudinal location relative to the final lead connector 20. The longitudinal spaces between steps can be adjusted and tailored to any particular application or standard to ensure proper spacing between rings. Similarly, first-fourth openings 68a, 68b, 68c, and 68d are configured to ensure that respective conductive pins 48a, 48b, 48c, and 48d are located properly to ensure conductive contact with appropriate rings and avoid inadvertent contact with the other rings and other conductors.

In one embodiment, first-third openings 68a, 68b, 68c, and thus, first-third conductive pins 48a, 48b, 48c, are arranged generally on the outer perimeter of assembly frame 60, such as viewed from the end illustrated in Figure 8. In one embodiment, conductive pins 48a, 48b, 48c are spaced apart around the perimeter to ensure no one conductor is too close to another, or too close to a ring to which it is not coupled.

In one embodiment, once first-third ring-pin subassemblies 38a/48a, 38b/48b, 38c/48c are individually placed on to assembly frame 60, the combined ring-pin assembly 70, illustrated in Figure 10, is then placed into a mold for over-molding to produce a completed lead connector 20, such as illustrated by Figures 1 and 2. Generally, the conductive pins and central pin/pin contact are positioned within the mold cavity with their ends extending through corresponding exit holes formed in the mold tool steel. A thermoplastic material, or other suitable material, is then injected under pressure into the cavity to over-mold the subassemblies and main contact pin to form the cylindrical body of the lead connector.

Figure 11 generally illustrates ring-pin assembly 70 placed in an injection molding system 80 for over-molding to form body 30, and thereby form completed lead connector 20. Molding system 80 includes a mold cavity 82 configured to receive a ring-pin assembly 70. According to one embodiment, mold cavity 82 is substantially tubular or cylindrical in shape, and has an inside diameter equal to that of the outside diameter of body 30 of lead connector 20. Mold cavity 82 includes an opening 84 at one end through which the proximal end of central pin 40, including pin contact 42, extends. Mold cavity 86 includes an opening 86 at an opposing end through which portions of the distal end of ring-pin assembly 70 extend, including the portions of conductive pins 38a-38c and central pin 40. Mold system 80 can also include a block 88 configured to retain a portion for connecting to pins 38a-38c.

In one embodiment, assembly frame 60 obviates the need for an assembly operator to carefully load each ring-pin subassembly into the mold cavity 82 and to carefully position them to ensure proper spacing and alignment. Traditional manufacturing process requires that each ring-pin subassembly must be nested and inserted individually into the mold tool, such that each ring and each conductor is precisely located to ensure proper spacing, thereby requiring skill and extra time during the assembly process.

After ring-pin assembly 70 is loaded into mold cavity 82, mold material is injected into mold cavity 82 to over-mold those portions of ring-pin assembly 70 within mold cavity 82 and form connector body 30. After removal from the mold cavity, assembly frame 60 is integral with body 30 and forms a portion of the finished lead connector 20.The resulting finished lead connector 20 (as illustrated by Figures 1 and 2) is then removed from mold system 80.

In one embodiment, assembly frame 60 is made of a material that is the same as the mold material injected into mold cavity 82. Since the mold material flows in hot, some or all portions of assembly frame 60 may melt and mix with the injected mold material. In other embodiments, assembly frame 60 is made of a material that is the different than the mold material injected into mold cavity 82. For example the materials may have different durometers and/or meting points, such that in some circumstances, the material of assembly frame 60 will remain relatively intact, but fully surrounded by the molding material in the final lead connector 20.

In one embodiment, the rigid structure of assembly frame 60, combined with a secure fit of first-third conductive pins 48a, 48b, 48c with first-third openings 68a, 68b, 68c, prevents the forces of mold material injected into the mold cavity 82 from moving and misaligning conductive pins 48a, 48b, 48c and rings 38a, 38b, 38c. In traditional manufacturing process, the ring-pin subassemblies are free-standing during molding. High injection pressure used in the injection molding process can cause the conductive pins and central pin to move within the mold cavity, thereby causing the electrical characteristics to potentially vary between lead connectors, and even causing shorting issues should the conductive pins be moved into contact with one another or other conductive elements within the lead connector. Forces created inside the mold cavity subject the pins to potential movement and bowing (forcing curvature) during the molding process. For example, during traditional manufacturing process, there are opportunities for electrical shorts or arcing between conductor paths. During the nesting process and the molding process it is imperative that the pin from one ring does not touch or come too near another ring or pin. Assembly frame 60 ensures such faults are avoided.

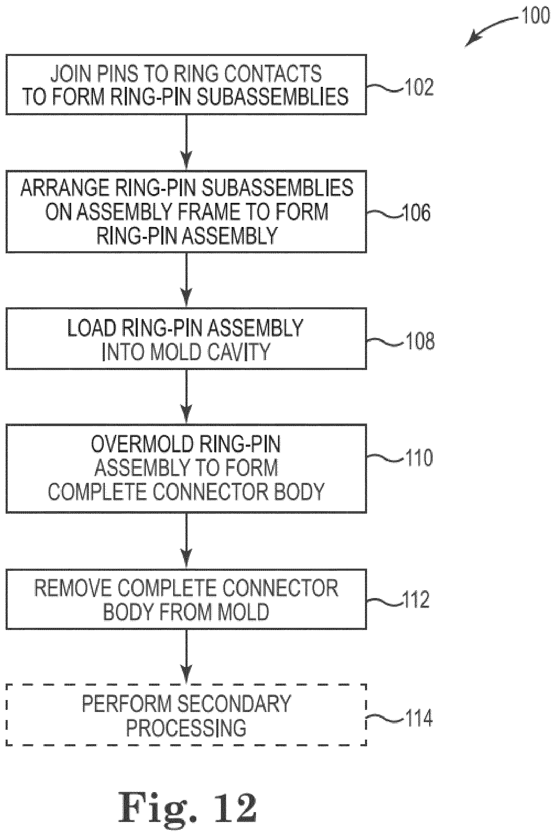

Figure 12 is flow diagram illustrating a process 100 for forming a lead connector, such as lead connector 20, according to one embodiment. Process 100 begins at 102 where conductive pins, such as conductive pins 48a-48c are joined (e.g. by laser welding) to ring contacts, such as ring contacts 38a-38c, to form ring-pin sub-assemblies.

At 106, the ring-pin sub-assemblies are arranged onto the assembly frame to form a ring-pin assembly. For example, according to one embodiment as illustrated by Figure 9, third ring-pin subassembly 38c/48c is arranged over assembly frame 60 by sliding third conductive pin 48c into third opening 68c and sliding third ring 38c over assembly frame 60 until it engages third step 62c, second ring-pin subassembly 38b/48b is arranged over assembly frame 60 by sliding second conductive pin 48b into second opening 68b and sliding second ring 38b over assembly frame 60 until it engages second step 62b, and first ring-pin subassembly 38a/48a is arranged over assembly frame 60 by sliding first conductive pin 48a into first opening 68a and sliding first ring 38a over assembly frame 60 until it engages first step 62a.

At 108, the ring-pin assembly, such as ring-pin assembly 70, is loaded into a mold cavity of an injection molding system, such as mold cavity 82 of molding system 80 (see Figure 11). Mold material is injected into the mold cavity to over-mold the portions of ring-pin assembly 70 within the mold cavity 82.

At 112, the finished lead connector, such as lead connector 20, is removed from the mold. At 114, if required, post mold secondary processing is performed, such as annealing, plasma treatment, machining, trimming or cleaning. For example, some thermoplastics require annealing in order to meet dimensional specification. Machining can be done to add a feature on an inner diameter or outer diameter of the lead connector that cannot be effectively formed via injection molding.

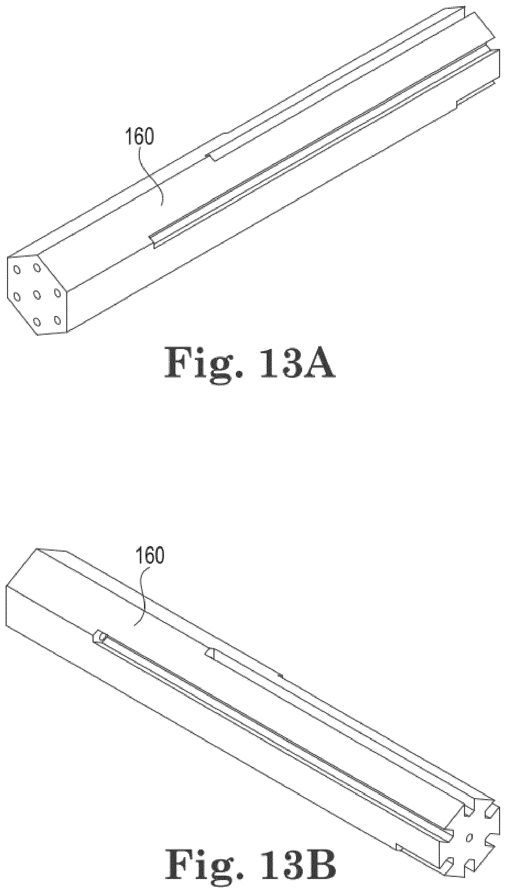

Figures 13A and 13B illustrate an assembly frame 160 in accordance with one embodiment. Assembly frame 160 is configured for use similar to that described above relative to assembly frame 60. For example, a plurality of ring-pin subassemblies, such as for example, first-third ring-pin subassemblies 38a/48a, 38b/48b, 38c/48c, can be arranged over assembly frame 160 to form a ring-pin assembly, which can then be overmolded to form a lead connector. Whereas assembly frame 60 illustrated in previous embodiments had a substantially square-shaped outer surface, especially when viewed from its distal end 34 (Figure 8), assembly frame 160 has an outer surface that is hexagonal. As is evident to one skilled in the art, an assembly frame can have an outer surface with any of a variety of shapes.

Also, whereas assembly frame 60 illustrated in previous embodiments had 3 openings around its perimeter and 3 steps spaced along its length, assembly frame 160 has 6 openings around its perimeter and 6 steps spaced along its length. In this way, assembly frame 160 can accommodate 6 ring-pin subassemblies, ensuring proper spacing between each ring and between each conductor in the ring-pin assemblies. Like assembly frame 60, assembly frame 160 can also be provided with a center opening to accommodate a center pin or other center conductor or the like. As is evident to one skilled in the art, an assembly frame can have any number of steps and openings to accommodate any number of ring-pin subassemblies.

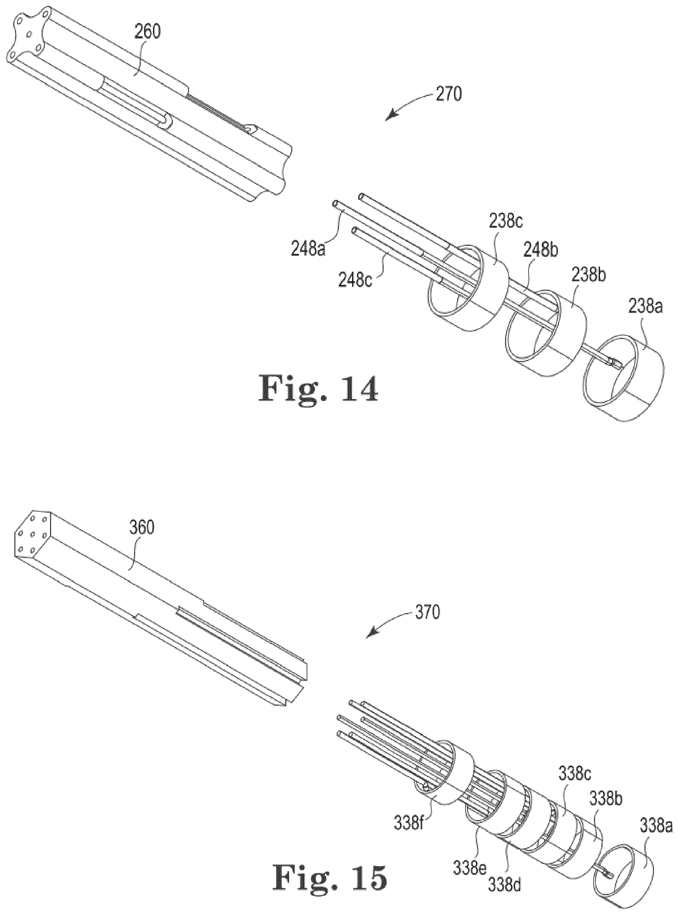

Figure 14 illustrates an exploded view of a partially assembled ring-pin assembly 270 for forming lead connector 20, where each of first-third ring-pin subassemblies 238a/248a, 238b/248b, 238c/248c are adjacent assembly frame 260. In one embodiment, third ring-pin subassembly 238c/248c is placed over assembly frame 260 by sliding third conductive pin 248c into third opening 268c and sliding third ring 238c over assembly frame 260 until it engages third step 262c. Next, second ring-pin subassembly 238b/248b is placed over assembly frame 260 by sliding second conductive pin 248b into second opening 268b and sliding second ring 238b over assembly frame 260 until it engages second step 262b. Finally, first ring-pin subassembly 238a/248a is placed over assembly frame 260 by sliding first conductive pin 248a into first opening 268a and sliding first ring 238a over assembly frame 260 until it engages first step 262a. In one embodiment, central pin 40 is inserted through fourth opening 268d so that it extends out of assembly frame 260 at both its ends. Once fully assembled, the ring-pin assembly is placed in a mold cavity and overmolded into a lead connector as previously described.

Figure 15 illustrates an exploded view of a partially assembled ring-pin assembly 370 for forming lead connector 20, where each of first-sixth ring-pin subassemblies 338a/348a, 338b/348b, 338c/348c, 338d/348d, 338e/348e, 338f/348f are adjacent assembly frame 360. In one embodiment, sixth ring-pin subassembly 338f/348f is placed over assembly frame 360 by sliding sixth conductive pin 348f into sixth opening 368f and sliding sixth ring 338f over assembly frame 360 until it engages sixth step 362f. Next, fifth ring-pin subassembly 338b/348e is placed over assembly frame 360 by sliding fifth conductive pin 348e into fifth opening 368e and sliding fifth ring 338e over assembly frame 360 until it engages fifth step 362e. Next, fourth ring-pin subassembly 338d/348d is placed over assembly frame 360 by sliding fourth conductive pin 348d into fourth opening 368d and sliding fourth ring 338d over assembly frame 360 until it engages fourth step 362d. Next, third ring-pin subassembly 338c/348c is placed over assembly frame 360 by sliding third conductive pin 348c into third opening 368c and sliding third ring 338c over assembly frame 360 until it engages third step 362c. Next, second ring-pin subassembly 338b/348b is placed over assembly frame 360 by sliding second conductive pin 348b into second opening 368b and sliding second ring 338b over assembly frame 360 until it engages second step 362b. Finally, first ring-pin subassembly 338a/348a is placed over assembly frame 360 by sliding first conductive pin 348a into first opening 368a and sliding first ring 338a over assembly frame 360 until it engages first step 362a.

In one embodiment, central pin 40 is inserted through seventh opening 268g so that it extends out of assembly frame 360 at both its ends. Once fully assembled, the ring-pin assembly is placed in a mold cavity and overmolded into a lead connector as previously described.

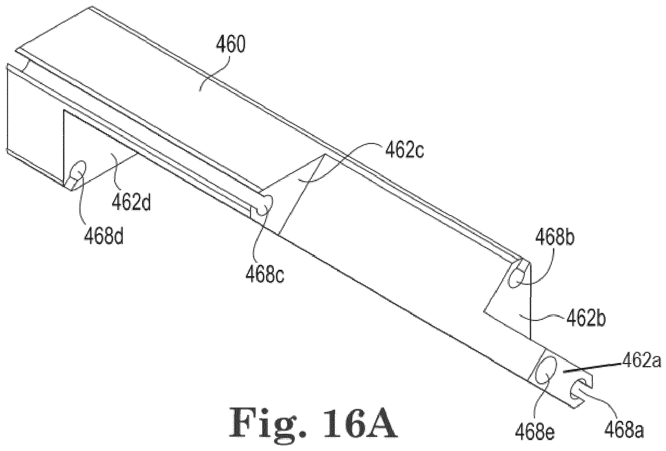

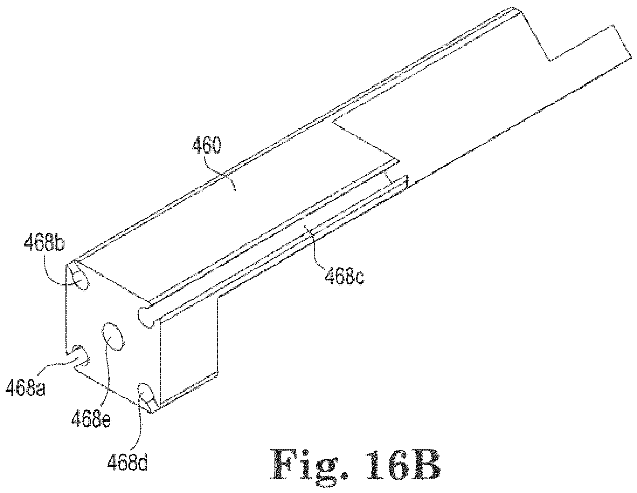

Figures 16A-16B illustrate assembly frame 460 in accordance with one embodiment. In one embodiment, assembly frame 460 is configured with first-fourth openings 468a, 468b, 468c, and 468d and with first-fourth steps 462a, 462b, 462c and 462d. First-fourth steps 462a, 462b, 462c and 462d are spaced along the longitudinal dimension of assembly frame 460 such that when a respective ring is placed adjacent the step, the ring will be situated in a desired longitudinal location relative to the final lead connector 20. The longitudinal spaces between steps can be adjusted and tailored to any particular application or standard to ensure proper spacing between rings. First-fourth openings 468a, 468b, 468c, and 468d are configured to ensure that respective conductive pins are located properly to ensure conductive contact with appropriate rings and avoid inadvertent contact with the other rings and other conductors. Assembly frame 460 also include fifth opening 468e though its center, which can in one embodiment accommodate a central pin.

In the illustrated assembly frame 460, first-fourth openings 468a, 468b, 468c, and 468d are configure as slots, with a longitudinally extending narrow slot-opening along the length of each opening. Configuring first-fourth openings 468a, 468b, 468c, and 468d as slots can be useful in assembling the pins and may have advantages in tooling in some embodiments. Once assembly frame 460 has respective conductive pins assembled into openings 468a, 468b, 468c, and 468d and rings assembled adjacent first-fourth steps 462a, 462b, 462c and 462d, it can be overmolded as described above for the other ring-pin assemblies, such that molded material will flow over and into the slots, completely surrounding the conductive pins.

Although specific examples have been illustrated and described herein, a variety of alternate and/or equivalent implementations may be substituted for the specific examples shown and described without departing from the scope of the present invention which is defined by the appended claims.

## Claims

1. A method of manufacturing a lead connector (20) for an implantable medical device, the method comprising:
connecting proximal ends (46a, 46b, and 46c) of a plurality of conductive pins (46a, 46b, 46c) to a corresponding one of a plurality of ring contacts (38a, 38b, 38c) to form a plurality of ring-pin subassemblies (38a/48a, 38b/48b, 38c/48c);
assembling each of the plurality of ring-pin subassemblies (38a/48a, 38b/48b, 38c/48c) on an assembly frame (60) to form a ring-pin assembly (70), including inserting the plurality of conductive pins (46a, 46b, 46c) in a corresponding plurality of openings (68a, 68b, 68c, 68d) within the assembly frame (60) such that the corresponding plurality of ring contacts (38a, 38b, 38c) are spaced along a longitudinal dimension of the assembly frame (60),
wherein the plurality of openings (68a, 68b, 68c, 68d) are configured as cylindrical lumens with a diameter that accommodates the plurality of conductive pins (46a, 46b, 46c),
wherein the assembly frame (60) comprises a plurality of steps (62a, 62b, 62c) and wherein assembling each of the plurality of ring-pin subassemblies (38a/48a, 38b/48b, 38c/48c) on the assembly frame (60) further comprises placing one of the plurality of ring contacts (38a, 38b, 38c) adjacent one of the plurality of steps (62a, 62b, 62c), and
wherein the plurality of openings (68a, 68b, 68c, 68d) within the assembly frame (60) are configured to ensure no conductive pin of the plurality of conductive pins (46a, 46b, 46c) contacts any other conductive pin of the plurality of conductive pins (46a, 46b, 46c);
arranging the ring-pin assembly (70) within a mold cavity (82);
filling the mold cavity (82) with a mold material that at least partially surrounds the ring-pin assembly (70); and
removing a resulting lead connector (20) from the mold cavity (82).

2. The method of any previous claim, wherein the plurality of openings (68a, 68b, 68c, and 68d) within the assembly frame (60) are configured to ensure each conductive pin of the plurality of conductive pins (46a, 46b, 46c) contacts only one ring contact of the plurality of ring contacts (38a, 38b, 38c).

3. The method of any previous claim, wherein the plurality of steps (62a, 62b, 62c) are spaced apart along a longitudinal dimension of the assembly frame (60) such that one of the plurality of steps (62a, 62b, 62c) corresponds to the location of one of the plurality of ring contacts (38a, 38b, 38c) of the lead connector (20).

4. The method of any previous claim, wherein the mold material filling the mold cavity (82) completely surrounds the assembly frame (60).

5. The method of any previous claim, wherein the mold material filling the mold cavity (82) is the same material as the material of the assembly frame (60).

6. A lead connector (20) for an implantable medical device, the lead connector (20) comprising:
a plurality of ring-pin subassemblies (38a/48a, 38b/48b, 38c/48c) comprising a plurality of conductive pins (46a, 46b, 46c) having proximal ends (46a, 46b, 46c) connecting to a corresponding one of a plurality of ring contacts (38a, 38b, 38c);
an assembly frame (60) comprising a plurality of openings (68a, 68b, 68c, 68d) and a plurality of steps (62a, 62b, 62c), wherein one of the plurality of conductive pins (46a, 46b, 46c) extends within one of the plurality of openings (68a, 68b, 68c, 68d), and wherein one of the plurality of ring contacts (38a, 38b, 38c) is adjacent one of the plurality of steps (62a, 62b, 62c),
wherein the plurality of openings (68a, 68b, 68c, 68d) are configured as cylindrical lumens with a diameter that accommodates the plurality of conductive pins (46a, 46b, 46c), and
wherein the plurality of openings (68a, 68b, 68c, 68d) within the assembly frame (60) are configured to ensure each conductive pin of the plurality of conductive pins (46a, 46b, 46c) contacts only one ring contact of the plurality of ring contacts (38a, 38b, 38c); and
an insulating material that at least partially surrounding the plurality of ring contacts (38a, 38b, 38c) and fully surrounding the assembly frame (60).

7. The lead connector (20) of any previous claim, wherein the plurality of openings (68a, 68b, 68c, 68d) within the assembly frame (60) are configured to ensure no conductive pin of the plurality of conductive pins (46a, 46b, 46c) contacts any other conductive pin of the plurality of conductive pins (46a, 46b, 46c).

8. The lead connector (20) of any previous claim, wherein the plurality of steps (62a, 62b, 62c) are spaced apart along a longitudinal dimension of the assembly frame (60) such that one of the plurality of steps (62a, 62b, 62c) corresponds to the location of one of the plurality of ring contacts (38a, 38b, 38c) of the lead connector (20).

9. The lead connector (20) of any previous claim, wherein the insulating material is the same material as the material of the assembly frame (60).

10. The lead connector (20) of any previous claim, wherein connecting proximal ends (46a, 46b, and 46c) of the plurality of conductive pins (46a, 46b, 46c) are coupled to an inner surface of a corresponding ring contact.

11. A ring-pin assembly (70) comprising:
a plurality of ring-pin subassemblies (38a/48a, 38b/48b, 38c/48c) comprising a plurality of conductive pins (46a, 46b, 46c) having proximal ends (46a, 46b, and 46c) connecting to a corresponding one of a plurality of ring contacts (38a, 38b, 38c);
an assembly frame (60) comprising a plurality of openings (68a, 68b, 68c, 68d) and a plurality of steps (62a, 62b, 62c), wherein one of the plurality of conductive pins (46a, 46b, 46c) extends within one of the plurality of openings (68a, 68b, 68c, 68d), and wherein one of the plurality of ring contacts (38a, 38b, 38c) is adjacent one of the plurality of steps (62a, 62b, 62c),
wherein the plurality of openings (68a, 68b, 68c, 68d) are configured as cylindrical lumens with a diameter that accommodates the plurality of conductive pins (46a, 46b, 46c), and
wherein the plurality of openings (68a, 68b, 68c, 68d) within the assembly frame (60) are configured to ensure each conductive pin of the plurality of conductive pins (46a, 46b, 46c) contacts only one ring contact of the plurality of ring contacts (38a, 38b, 38c).

12. The ring-pin assembly (70) of any previous claim, wherein an insulating material at least partially surrounds the plurality of ring contacts (38a, 38b, 38c) and fully surrounds the assembly frame (60), thereby forming a lead connector (20).

## Patentansprüche

1. Verfahren zum Herstellen eines Leitungsverbinders (20) für eine implantierbare medizinische Vorrichtung, das Verfahren umfassend:
Verbinden von proximalen Enden (46a, 46b und 46c) einer Vielzahl von leitenden Stiften (46a, 46b, 46c) mit einem entsprechenden einer Vielzahl von Ringkontakten (38a, 38b, 38c), um eine Vielzahl von Ring-Stift-Unterbaugruppen (38a/48a, 38b/48b, 38c/48c) auszubilden;
Montieren jeder der Vielzahl von Ring-Stift-Unterbaugruppen (38a/48a, 38b/48b, 38c/48c) auf einem Montagerahmen (60), um eine Ring-Stift-Baugruppe (70) auszubilden, einschließlich eines Einsetzens der Vielzahl von leitenden Stiften (46a, 46b, 46c) in eine entsprechende Vielzahl von Öffnungen (68a, 68b, 68c, 68d) innerhalb des Montagerahmens (60), derart, dass die entsprechende Vielzahl von Ringkontakten (38a, 38b, 38c) entlang einer Längsabmessung des Montagerahmens (60) beabstandet sind,
wobei die Vielzahl von Öffnungen (68a, 68b, 68c, 68d) als zylindrische Lumen mit einem Durchmesser konfiguriert sind, der die Vielzahl von leitenden Stiften (46a, 46b, 46c) aufnimmt,
wobei der Montagerahmen (60) eine Vielzahl von Stufen (62a, 62b, 62c) umfasst, und wobei das Montieren jeder der Vielzahl von Ring-Stift-Unterbaugruppen (38a/48a, 38b/48b, 38c/48c) auf dem Montagerahmen (60) ferner ein Platzieren eines der Vielzahl von Ringkontakten (38a, 38b, 38c) angrenzend an eine der Vielzahl von Stufen (62a, 62b, 62c) umfasst, und
wobei die Vielzahl von Öffnungen (68a, 68b, 68c, 68d) innerhalb des Montagerahmens (60) konfiguriert sind, um sicherzustellen, dass kein leitender Stift der Vielzahl von leitenden Stiften (46a, 46b, 46c) mit einem beliebigen anderen leitenden Stift der Vielzahl von leitenden Stiften (46a, 46b, 46c) in Kontakt kommt;
Anordnen der Ring-Stift-Baugruppe (70) innerhalb eines Formhohlraums (82);
Füllen des Formhohlraums (82) mit einem Formmaterial, das die Ring-Stift-Baugruppe (70) mindestens teilweise umgibt; und
Entfernen eines resultierenden Leitungsverbinders (20) aus dem Formhohlraum (82).

2. Verfahren nach einem der vorstehenden Ansprüche, wobei die Vielzahl von Öffnungen (68a, 68b, 68c und 68d) innerhalb des Montagerahmens (60) konfiguriert sind, um sicherzustellen, dass jeder leitende Stift der Vielzahl von leitenden Stiften (46a, 46b, 46c) nur mit einem Ringkontakt der Vielzahl von Ringkontakten (38a, 38b, 38c) in Kontakt kommt.

3. Verfahren nach einem der vorstehenden Ansprüche, wobei die Vielzahl von Stufen (62a, 62b, 62c) entlang einer Längsabmessung des Montagerahmens (60) derart beabstandet sind, dass eine der Vielzahl von Stufen (62a, 62b, 62c) der Stelle eines der Vielzahl von Ringkontakten (38a, 38b, 38c) des Leitungsverbinders (20) entspricht.

4. Verfahren nach einem der vorstehenden Ansprüche, wobei das Formmaterial, das den Formhohlraum (82) füllt, den Montagerahmen (60) vollständig umgibt.

5. Verfahren nach einem der vorstehenden Ansprüche, wobei das Formmaterial, das den Formhohlraum (82) füllt, das gleiche Material wie das Material des Montagerahmens (60) ist.

6. Leitungsverbinder (20) für eine implantierbare medizinische Vorrichtung, der
Leitungsverbinder (20) umfassend:
eine Vielzahl von Ring-Stift-Unterbaugruppen (38a/48a, 38b/48b, 38c/48c), umfassend eine Vielzahl von leitenden Stiften (46a, 46b, 46c), die proximale Enden (46a, 46b, 46c) aufweisen, die mit einem entsprechenden einer Vielzahl von Ringkontakten (38a, 38b, 38c) verbunden sind;
einen Montagerahmen (60), umfassend eine Vielzahl von Öffnungen (68a, 68b, 68c, 68d) und eine Vielzahl von Stufen (62a, 62b, 62c), wobei sich einer der Vielzahl von leitenden Stiften (46a, 46b, 46c) innerhalb einer der Vielzahl von Öffnungen (68a, 68b, 68c, 68d) erstreckt, und wobei einer der Vielzahl von Ringkontakten (38a, 38b, 38c) angrenzend an eine der Vielzahl von Stufen (62a, 62b, 62c) ist,
wobei die Vielzahl von Öffnungen (68a, 68b, 68c, 68d) als zylindrische Lumen mit einem Durchmesser konfiguriert sind, der die Vielzahl von leitenden Stiften (46a, 46b, 46c) aufnimmt, und
wobei die Vielzahl von Öffnungen (68a, 68b, 68c, 68d) innerhalb des Montagerahmens (60) konfiguriert sind, um sicherzustellen, dass jeder leitende Stift der Vielzahl von leitenden Stiften (46a, 46b, 46c) nur mit einem Ringkontakt der Vielzahl von Ringkontakten (38a, 38b, 38c) in Kontakt kommt; und
ein Isoliermaterial, das die Vielzahl von Ringkontakten (38a, 38b, 38c) mindestens teilweise umgibt und den Montagerahmen (60) vollständig umgibt.

7. Leitungsverbinder (20) nach einem der vorstehenden Ansprüche, wobei die Vielzahl von Öffnungen (68a, 68b, 68c, 68d) innerhalb des Montagerahmens (60) konfiguriert sind, um sicherzustellen, dass kein leitender Stift der Vielzahl von leitenden Stiften (46a, 46b, 46c) mit einem beliebigen anderen leitenden Stift der Vielzahl von leitenden Stiften (46a, 46b, 46c) in Kontakt kommt.

8. Leitungsverbinder (20) nach einem der vorstehenden Ansprüche, wobei die Vielzahl von Stufen (62a, 62b, 62c) entlang einer Längsabmessung des Montagerahmens (60) derart beabstandet sind, dass eine der Vielzahl von Stufen (62a, 62b, 62c) der Stelle eines der Vielzahl von Ringkontakten (38a, 38b, 38c) des Leitungsverbinders (20) entspricht.

9. Leitungsverbinder (20) nach einem der vorstehenden Ansprüche, wobei das Isoliermaterial das gleiche Material wie das Material des Montagerahmens (60) ist.

10. Leitungsverbinder (20) nach einem der vorstehenden Ansprüche, wobei die verbindenden proximalen Enden (46a, 46b und 46c) der Vielzahl von leitenden Stiften (46a, 46b, 46c) mit einer Innenoberfläche eines entsprechenden Ringkontakts gekoppelt sind.

11. Ring-Stift-Baugruppe (70), umfassend:
eine Vielzahl von Ring-Stift-Unterbaugruppen (38a/48a, 38b/48b, 38c/48c), umfassend eine Vielzahl von leitenden Stiften (46a, 46b, 46c), die proximale Enden (46a, 46b und 46c) aufweisen, die mit einem entsprechenden einer Vielzahl von Ringkontakten (38a, 38b, 38c) verbunden sind;
einen Montagerahmen (60), umfassend eine Vielzahl von Öffnungen (68a, 68b, 68c, 68d) und eine Vielzahl von Stufen (62a, 62b, 62c), wobei sich einer der Vielzahl von leitenden Stiften (46a, 46b, 46c) innerhalb einer der Vielzahl von Öffnungen (68a, 68b, 68c, 68d) erstreckt, und wobei einer der Vielzahl von Ringkontakten (38a, 38b, 38c) angrenzend an eine der Vielzahl von Stufen (62a, 62b, 62c) ist,
wobei die Vielzahl von Öffnungen (68a, 68b, 68c, 68d) als zylindrische Lumen mit einem Durchmesser konfiguriert sind, der die Vielzahl von leitenden Stiften (46a, 46b, 46c) aufnimmt, und
wobei die Vielzahl von Öffnungen (68a, 68b, 68c, 68d) innerhalb des Montagerahmens (60) konfiguriert sind, um sicherzustellen, dass jeder leitende Stift der Vielzahl von leitenden Stiften (46a, 46b, 46c) nur mit einem Ringkontakt der Vielzahl von Ringkontakten (38a, 38b, 38c) in Kontakt kommt.

12. Ring-Stift-Baugruppe (70) nach einem der vorstehenden Ansprüche, wobei ein Isoliermaterial die Vielzahl der Ringkontakte (38a, 38b, 38c) mindestens teilweise umgibt und den Montagerahmen (60) vollständig umgibt, wodurch ein Leitungsverbinder (20) ausgebildet wird.

## Revendications

1. Procédé de fabrication d'un connecteur de dérivation (20) destiné à un dispositif médical implantable, le procédé comprenant :
la liaison d'extrémités proximales (46a, 46b et 46c) d'une pluralité de broches conductrices (46a, 46b, 46c) à l'un correspondant parmi une pluralité de contacts annulaires (38a, 38b, 38c) pour former une pluralité de sous-ensembles anneau-broche (38a/48a, 38b/48b, 38c/48c) ;
l'assemblage de chacun parmi la pluralité de sous-ensembles anneau-broche (38a/48a, 38b/48b, 38c/48c) sur un bâti d'assemblage (60) pour former un ensemble anneau-broche (70), comportant l'insertion de la pluralité de broches conductrices (46a, 46b, 46c) dans une pluralité correspondante d'ouvertures (68a, 68b, 68c, 68d) au sein du bâti d'assemblage (60) de telle sorte que la pluralité correspondante de contacts annulaires (38a, 38b, 38c) sont espacés le long d'une dimension longitudinale du bâti d'assemblage (60), dans lequel la pluralité d'ouvertures (68a, 68b, 68c, 68d) sont conçues en tant que lumières cylindriques avec un diamètre qui accueille la pluralité de broches conductrices (46a, 46b, 46c),
dans lequel le bâti d'assemblage (60) comprend une pluralité d'étages (62a, 62b, 62c) et dans lequel l'assemblage de chacun parmi la pluralité de sous-ensembles anneau-broche (38a/48a, 38b/48b, 38c/48c) sur le bâti d'assemblage (60) comprend en outre la mise en place de l'un parmi la pluralité de contacts annulaires (38a, 38b, 38c) de manière adjacente à l'un parmi la pluralité d'étages (62a, 62b, 62c), et
dans lequel la pluralité d'ouvertures (68a, 68b, 68c, 68d) au sein du bâti d'assemblage (60) sont conçues pour garantir qu'aucune broche conductrice de la pluralité de broches conductrices (46a, 46b, 46c) ne vient en contact avec l'une quelconque autre broche conductrice de la pluralité de broches conductrices (46a, 46b, 46c) ;
l'agencement de l'ensemble anneau-broche (70) au sein d'une cavité de moule (82) ;
le remplissage de la cavité de moule (82) d'un matériau de moule qui entoure au moins partiellement l'ensemble anneau-broche (70) ; et
le retrait d'un connecteur de dérivation (20) résultant de la cavité de moule (82).

2. Procédé selon l'une quelconque revendication précédente, dans lequel la pluralité d'ouvertures (68a, 68b, 68c et 68d) au sein du bâti d'assemblage (60) sont conçues pour garantir que chaque broche conductrice de la pluralité de broches conductrices (46a, 46b, 46c) ne vient en contact qu'avec un seul contact annulaire de la pluralité de contacts annulaires (38a, 38b, 38c).

3. Procédé selon l'une quelconque revendication précédente, dans lequel la pluralité d'étages (62a, 62b, 62c) sont espacés le long d'une dimension longitudinale du bâti d'assemblage (60) de telle sorte que l'un parmi la pluralité d'étages (62a, 62b, 62c) correspond à l'emplacement de l'un parmi la pluralité de contacts annulaires (38a, 38b, 38c) du connecteur de dérivation (20).

4. Procédé selon l'une quelconque revendication précédente, dans lequel le matériau de moule remplissant la cavité de moule (82) entoure complètement le bâti d'assemblage (60).

5. Procédé selon l'une quelconque revendication précédente, dans lequel le matériau de moule remplissant la cavité de moule (82) est le même matériau que le matériau du bâti d'assemblage (60).

6. Connecteur de dérivation (20) destiné à un dispositif médical implantable, le connecteur de dérivation (20) comprenant :
une pluralité de sous-ensembles anneau-broche (38a/48a, 38b/48b, 38c/48c) comprenant une pluralité de broches conductrices (46a, 46b, 46c) ayant des extrémités proximales (46a, 46b, 46c) se reliant à l'un correspondant parmi une pluralité de contacts annulaires (38a, 38b, 38c) ;
un bâti d'assemblage (60) comprenant une pluralité d'ouvertures (68a, 68b, 68c, 68d) et une pluralité d'étages (62a, 62b, 62c), dans lequel l'une parmi la pluralité de broches conductrices (46a, 46b, 46c) s'étend au sein de l'une parmi la pluralité d'ouvertures (68a, 68b, 68c, 68d), et dans lequel l'un parmi la pluralité de contacts annulaires (38a, 38b, 38c) est adjacent à l'un parmi la pluralité d'étages (62a, 62b, 62c),
dans lequel la pluralité d'ouvertures (68a, 68b, 68c, 68d) sont conçues en tant que lumières cylindriques avec un diamètre qui accueille la pluralité de broches conductrices (46a, 46b, 46c), et
dans lequel la pluralité d'ouvertures (68a, 68b, 68c, 68d) au sein du bâti d'assemblage (60) sont conçues pour garantir que chaque broche conductrice de la pluralité de broches conductrices (46a, 46b, 46c) ne vient en contact qu'avec un seul contact annulaire de la pluralité de contacts annulaires (38a, 38b, 38c) ; et
un matériau isolant qui entoure au moins partiellement la pluralité de contacts annulaires (38a, 38b, 38c) et entoure complètement le bâti d'assemblage (60).

7. Connecteur de dérivation (20) selon l'une quelconque revendication précédente, dans lequel la pluralité d'ouvertures (68a, 68b, 68c, 68d) au sein du bâti d'assemblage (60) sont conçues pour garantir qu'aucune broche conductrice de la pluralité de broches conductrices (46a, 46b, 46c) ne vient en contact avec l'une quelconque autre broche conductrice de la pluralité de broches conductrices (46a, 46b, 46c).

8. Connecteur de dérivation (20) selon l'une quelconque revendication précédente, dans lequel la pluralité d'étages (62a, 62b, 62c) sont espacés le long d'une dimension longitudinale du bâti d'assemblage (60) de telle sorte que l'un parmi la pluralité d'étages (62a, 62b, 62c) correspond à l'emplacement de l'un parmi la pluralité de contacts annulaires (38a, 38b, 38c) du connecteur de dérivation (20).

9. Connecteur de dérivation (20) selon l'une quelconque revendication précédente, dans lequel le matériau isolant est le même matériau que le matériau du bâti d'assemblage (60).

10. Connecteur de dérivation (20) selon l'une quelconque revendication précédente, dans lequel les extrémités proximales (46a, 46b et 46c) de liaison de la pluralité de broches conductrices (46a, 46b, 46c) sont accouplées à une surface interne d'un contact annulaire correspondant.

11. Ensemble anneau-broche (70) comprenant :
une pluralité de sous-ensembles anneau-broche (38a/48a, 38b/48b, 38c/48c) comprenant une pluralité de broches conductrices (46a, 46b, 46c) ayant des extrémités proximales (46a, 46b et 46c) se reliant à l'un correspondant parmi une pluralité de contacts annulaires (38a, 38b, 38c) ;
un bâti d'assemblage (60) comprenant une pluralité d'ouvertures (68a, 68b, 68c, 68d) et une pluralité d'étages (62a, 62b, 62c), dans lequel l'une parmi la pluralité de broches conductrices (46a, 46b, 46c) s'étend au sein de l'une parmi la pluralité d'ouvertures (68a, 68b, 68c, 68d), et dans lequel l'un parmi la pluralité de contacts annulaires (38a, 38b, 38c) est adjacent à l'un parmi la pluralité d'étages (62a, 62b, 62c),
dans lequel la pluralité d'ouvertures (68a, 68b, 68c, 68d) sont conçues en tant que lumières cylindriques avec un diamètre qui accueille la pluralité de broches conductrices (46a, 46b, 46c), et
dans lequel la pluralité d'ouvertures (68a, 68b, 68c, 68d) au sein du bâti d'assemblage (60) sont conçues pour garantir que chaque broche conductrice de la pluralité de broches conductrices (46a, 46b, 46c) ne vient en contact qu'avec un seul contact annulaire de la pluralité de contacts annulaires (38a, 38b, 38c).

12. Ensemble anneau-broche (70) selon l'une quelconque revendication précédente, dans lequel un matériau isolant entoure au moins partiellement la pluralité de contacts annulaires (38a, 38b, 38c) et entoure complètement le bâti d'assemblage (60), en formant de ce fait un connecteur de dérivation (20).
